# EUROPEAN PATENT SPECIFICATION

(11) **EP 0 654 263 B1**
(45) Date of publication and mention of the grant of the patent: **23.01.2002**
(21) Application number: 94308493.9
(22) Date of filing: 17.11.1994
(51) Int. Cl.: A61K 31/135, A61K 31/485, A61K 9/16

(54) **Method for preparing a sustained release composition**
Verfahren zur Herstellung einer Arzneizusammensetzung mit verzögerter Wirkstoffabgabe
Procédé pour la préparation d'une composition pharmaceutique à libération prolongée

(30) Priority: 23.11.1993 GB 9324045; 01.03.1994 GB 9403922; 09.03.1994 GB 9404544; 14.03.1994 GB 9404928; 14.06.1994 GB 9411842; 09.06.1994 EP 94304144; 29.04.1994 EP 94303128
(43) Date of publication of application: 24.05.1995
(73) Proprietor: Euro-Celtique S.A., Luxemburg (LU)
(72) Inventor: Miller, Ronald Brown, Basle, 4051 (CH); Leslie, Stewart Thomas, Cambridge (GB); Malkowska, Sandra Therese Antoinette, Ely, Cambridge (GB); Prater, Derek Allan, Milton, Cambridge (GB); Knott, Trevor John, Wickford, Essex (GB); Heafield, Joanne, Fenstanton, Cambridge (GB); Challis, Deborah, Tunbridge, Kent, TN11 9LU (GB)
(74) Representative: Ruffles, Graham Keith

(56) References cited:
- WO-A-92/06679
- WO-A-93/18753
- US-A- 4 132 753

## Description

The present invention relates generally to a method of manufacturing pharmaceutical dosage forms, for human or veterinary use, preferably sustained release particles, such particles having diameters ranging from 0.1 to 3.0mm. Such particles may contain analgesics, such as morphine, or other active ingredients. The present invention also relates to dosage forms obtained by processing of the aforesaid particles, such as tablets, suppositories or pessaries.

Patent Application PCT/SE93/00225 published under No. WO 93/18753 describes a process for the preparation of sustained release pellets which comprises pelletising a mixture containing the drug in finely divided form and a binder; the process is characterised in that:
(a) the binder is in particle form consisting of one or more water-insoluble or water-soluble, wax-like binder substance(s) with a melting point above 40°C and
(b) the pelletisation step is performed by mechanically working the mixture, in a so-called high-shear mixer, under the input of a sufficient amount of energy for the binder to melt and pelletisation to take place.

Patent Application PCT/SE91/0069 published under No. WO 92/06679 describes a similar process.

Processes of this kind are sometimes referred to as "melt-pelletisation" processes. We have found that operating according to these processes using commercial manufacturing equipment with a standard stainless steel interior, which is also the method described in Schaefer et al. (Drug Development and Industrial Pharmacy, 16(8), 1249-1277 (1990) and Taggart et al. (International Journal of Pharmaceutics 19 (1984) 139-148), results in yields of pellets in the preferred size range of only about 30 to 60% compared with the theoretical. Use of a wider particle size range to improve the yield results in an erratic in vitro release rate and irreproducible performance.

There is, therefore, a need for a commercial process for producing satisfactory controlled release particles which has a much higher yield. One object of the invention is, therefore, to provide a process which has an improved yield and preferably produces a product with reproducible controlled release characteristics.

U.S. 4,132,753 describes a process of preparing granules of controlled release medicament by continually agitating a mixture of powdered medicament and finely divided wax-like material while subjecting the mixture to radiation heating.

The present invention provides a process for the manufacture of sustained release particles, which comprises
(a) mechanically working in a high-speed mixer, a mixture of a particulate drug and a particulate, hydrophobic and/or hydrophilic fusible carrier or diluent having a melting point from 35 to 150°C and optionally a release control component comprising a water-soluble fusible material or a particulate, soluble or insoluble organic or inorganic material, at a speed and energy input which allows the carrier or diluent to melt or soften whereby it forms agglomerates;
(b) removing agglomerates from the mixer and comminuting them to give controlled release particles; and optionally
(c) continuing mechanically working optionally with the addition of carrier or diluent in an amount of between 5% and 75% w/w of the total amount of ingredients; and optionally
(d) repeating steps (c) and possibly (b) one or more, e.g. up to five, times.

This process is capable of giving a high yield (over 80%) of particles in a desired size range, with a desired in vitro release rate and, uniformity of release rate.

The resulting particles may be sieved to eliminate any oversized or undersized material then formed into the desired dosage units by for example, encapsulation into hard gelatin capsules containing the required dose of the active substance or by tabletting, filling into sachets or moulding into suppositories, pessaries or forming into other suitable dosage forms.

The drug may be water soluble or water insoluble. Water soluble drugs will usually be used in amounts giving for example a loading of up to about 90% wlw in the resulting particles; water insoluble drugs may be used in higher amounts eg. up to 99% wlw of the resulting particles; Examples of water soluble drugs which can be used in the method of the invention are morphine, hydromorphone, diltiazem, diamorphine and tramadol and pharmaceutically acceptable salts thereof; examples of water insoluble drugs which can be used in the process of the invention are naproxen, ibuprofen, indomethacin and nifedipine.

Among the active ingredients which can be used in the process of the invention are the following;

### ANALGESICS

Dihydrocodeine, Hydromorphone, Morphine, Diamorphine, Fentanyl, Alflentanil, Sufentanyl, Pentazocine, Buprenorphine, Nefopam, Dextropropoxyphene, Flupirtine, Tramadol, Oxycodone, Metamizol, Propyphenazone, Phenazone, Nifenazone, Paracetamol, Phenylbutazone, Oxyphenbutazone, Mofebutazone, Acetyl salicylic acid, Diflunisal, Flurbiprofen, Ibuprofen, Diclofenac, Ketoprofen, Indomethacin, Naproxen, Meptazinol, Methadone, Pethidine, Hydrocodone, Meloxicam, Fenbufen, Mefenamic acid, Piroxicam, Tenoxicam, Azapropazone, Codeine,

### ANTIALLERGICS

Pheniramine, Dimethindene, Terfenadine, Astemizole, Tritoqualine, Loratadine, Doxylamine, Mequitazine, Dexchlorpheniramine, Triprolidine, Oxatomide,

### ANTIHYPERTENSIVE

Clonidine, Moxonidine, Methyldopa, Doxazosin, Prazosin, Urapidil, Terazosin, Minoxidil, Dihydralazin, Deserpidine, Acebutalol, Alprenolol, Atenolol, Metoprolol, Bupranolol, Penbutolol, Propranolol, Esmolol, Bisoprolol, Ciliprolol, Sotalol, Metipranolol, Nadolol, Oxprenolol, Nifedipine, Nicadipine, Verapamil, Diltiazem, Felodipine, Nimodipine, Flunarizine, Quinapril, Lisinopril, Captopril, Ramipril, Fosinopril, Cilazapril, Enalapril.

### ANTIBIOTICS

Democlocycline, Doxycycline, Lymecycline, Minocycline, Oxytetracycline, Tetracycline, Sulfametopyrazine, Ofloxacin, Ciproflaxacin, Aerosoxacin, Amoxycillin, Ampicillin, Becampicillin, Piperacillin, Pivampicillin, Cloxacillin, Penicillin V, Flucloxacillin, Erythromycin, Metronidazole, Clindamycin, Trimethoprim, Neomycin, Cefaclor, Cefadroxil, Cefixime, Cefpodoxime, Cefuroxine, Cephalexin, Cefradine.

### BRONCHODILATOR/ANTI-ASTHMATIC

Pirbuterol, Orciprenaline, Terbutaline, Fenoterol, Clenbuterol, Salbutamol, Procaterol, Theophylline, Cholintheophyllinate, Theophylline-ethylenediamine, Ketofen,

### ANTIARRHYTHMICS

Viquidil, Procainamide, Mexiletine, Tocainide, Propafenone, Ipratropium,

### CENTRALLY ACTING SUBSTANCES

Amantadine, Levodopa, Biperiden, Benzotropine, Bromocriptine, Procyclidine, Moclobemide, Tranylcypromide, Clomipramine, Maprotiline, Doxepin, Opipramol, Amitriptyline, Desipramine, Imipramine, Fluroxamin, Fluoxetin, Paroxetine, Trazodone, Viloxazine, Fluphenazine, Perphenazine, Promethazine, Thioridazine, Triflupromazine, Prothipendyl, Tiotixene, Chlorprothixene, Haloperidol, Pipamperone, Pimozide, Sulpiride, Fenethylline, Methylphenildat, Trifluoperazine, Thioridazine, Oxazepam, Lorazepam, Bromoazepam, Alprazolam, Diazepam, Clobazam, Buspirone, Piracetam,

### CYTOSTATICS AND METASTASIS INHIBITORS

Melfalan, Cyclophosphamide, Trofosfamide, Chlorambucil, Lomustine, Busulfan, Prednimustine, Fluorouracil, Methotrexate, Mercaptopurine, Thioguanin, Hydroxycarbamide, Altretamine, Procarbazine,

### ANTI-MIGRAINE

Lisuride, Methysergide, Dihydroergotamine, Ergotamine, Pizotifen,

### GASTROINTESTINAL

Cimetidine, Famotidine, Ranitidine, Roxatidine, Pirenzipine, Omeprazole, Misoprostol, Proglumide, Cisapride, Bromopride, Metoclopramide,

### ORAL ANTIDIABETICS

Tobutamide, Glibenclamide, Glipizide, Gliquidone, Gliboruride, Tolazamide, Acarbose and the pharmaceutically active salts or esters of the above and combinations of two or more of the above or salts or esters thereof.

The hydrolysis of drugs constitutes the most frequent, and perhaps therefore the most important, route of drug decomposition. Analysis of a collection of stability data in Connors KA, Amidon GL, Stella VJ, Chemical stability of pharmaceuticals. A handbook for pharmacists, 2nd ed. New York: John Wiley & Sons, 1986, a standard text, shows that over 70% of the drugs studied undergo hydrolytic degradation reactions. Of these, 61.4% can be classed as reactions of carboxylic acid derivatives (esters, amides, thiol esters, lactams, imides), 20% of carbonyl derivatives (imines, oximes) 14.3 % of nucleophilic displacements, and 4.3% of phosphoric acid derivatives. Cephalosporins, penicillins and barbiturates are particularly susceptible drug classes.

The process of the invention may advantageously be used for preparing dosage forms containing active substances as mentioned above which are unstable in the presence of water, e.g. diamorphine. Thus stable formulations of such drugs having normal or controlled release characteristics can be obtained in accordance with the invention.

In a preferred method according to the invention morphine sulphate, or other water soluble drug, e.g. tramadol, is used in an amount which results in particles containing e.g. between <1% and 90%, especially between about 45% and about 75% wlw active ingredient for a high dose product and e.g. < 1 and 45% for a low dose product.

In the method of the invention preferably all the drug is added in step (a) together with a major portion of the hydrophobic or hydrophilic fusible carrier or diluent used. Preferably the amount of fusible carrier or diluent added in step (a) is between e.g. 10% and <99% w/w of the total amount of ingredients added in the entire manufacturing operation.

The fusible carrier or diluent may be added stepwise during mechanical working, in step a) or step c).

Stage (a) of the process may be carried out in conventional high speed mixers with a standard stainless steel interior, e.g. a Collette Vactron 75 or equivalent mixer. The mixture is processed until a bed temperature above 40°C is achieved and the resulting mixture acquires a cohesive granular texture, with particle sizes ranging from about 1-3 mm to fine powder in the case of non-aggregated original material. Such material, in the case of the embodiments described below, has the appearance of agglomerates which upon cooling below 40°C have structural integrity and resistance to crushing between the fingers. At this stage the agglomerates are of an irregular size, shape and appearance.

The agglomerates are preferably allowed to cool. The temperature to which it cools is not critical and a temperature in the range room temperature to 41°C may be conveniently used.

The agglomerates are broken down by any suitable means, which will comminute oversize agglomerates and produce a mixture of powder and small particles preferably with a diameter under 2mm. It is currently preferred to carry out the classification using a Jackson Crockatt granulator using a suitable sized mesh, or a Comil with an appropriate sized screen. We have found that if too small a mesh size is used in the aforementioned apparatus the agglomerates melting under the action of the beater or impeller will clog the mesh and prevent further throughput of mixture, thus reducing yield.

The classified material is preferably returned to the high speed mixer and processing continued. It is believed that this leads to cementation of the finer particles into particles of uniform size range.

In one preferred form of the process of the invention processing of the classified materials is continued, until the hydrophobic and/or hydrophilic fusible carrier or diluent materials used begin to soften/melt and additional hydrophobic and/or hydrophilic fusible carrier or diluent material is then added; most preferably the additional hydrophobic and/or hydrophilic fusible carrier or diluent material is added after any fines generated in stage (b) have been taken up by the larger sized particles. Mixing is continued until the mixture has been transformed into particles of the desired predetermined size range.

In order to ensure uniform energy input into the ingredients in the high speed mixer it is preferred to supply at least part of the energy by means of microwave energy.

Energy may also be delivered through other means such as by a heating jacket or via the mixer impeller and chopper blades.

After the particles have been formed they are sieved to remove any oversized or undersized material and then cooled or allowed to cool.

The resulting particles may be used to prepare dosage units e.g. tablets or capsules in manners known per se.

In this process of the invention the temperature of the mixing bowl throughout the mechanical working is chosen so as to avoid excessive adhesion, suitably to minimise adhesion of the material to the walls of the bowl. To minimise adhesion we have generally found that the temperature should be neither too high nor too low with respect to the melting temperature of the material and it can be readily optimised to avoid the problems mentioned above. For example in the processes described below in the Examples a bowl temperature of approximately 50 - 60°C has been found to be satisfactory and avoids adhesion to the bowl. It is not possible to generalize as to the appropriate temperature for any particular mixture to be processed. However, in practice, it is a matter of simple experimentation and observation to establish a suitable temperature and processing time for a particular mixture under consideration.

The process of the invention described above is capable, in a preferred form, of providing particles which function as sustained release dosage forms. In particular, as described in co-pending European Patent Application No. 94303128.6 filed on 29th April 1994, an orally administrable sustained release dosage unit form containing morphine, or a pharmaceutically acceptable salt thereof, as active ingredient which formulation has a peak plasma level of morphine from 1 to 6 hours after administration.

We have found that by suitable selection of the materials used in forming the particles and in the tabletting and the proportions in which they are used, enables a significant degree of control in the ultimate dissolution and release rates of the active ingredients from the compressed tablets.

Suitable substances for use as hydrophobic carrier or diluent materials are natural or synthetic waxes or oils, for example hydrogenated vegetable oil, hydrogenated castor oil, Beeswax, Carauba wax, microcrystalline wax and glycerol monostearate, and suitably have melting points of from 35 to 150°C, preferably 45 to 90°C.

Suitable substances for use as hydrophillic carrier or diluent materials are natural or synthetic waxes or oils, for example PEGs having molecular weights of 1000 to 20,000 e.g. 1000 to 6000 or 10000 suitably having melting points of from 35 to 150°C, preferably 45 to 90°C.

The optionally added release control component when a water soluble, fusible material may be a PEG of appropriate molecular weight; suitable particulate inorganic and organic materials are, for example dicalcium phosphate, calcium sulphate, talc, colloidal anhydrous silica, and lactose, poloxamers, microcrystalline cellulose, starch, hydroxypropylcellulose, and hydroxypropylmethylcellulose.

To produce tablets in accordance with the process of the invention, particles produced as described above may be mixed or blended with the desired excipient(s), if any, using conventional procedures e.g. using a Y-Cone or bin-blender and the resulting mixture compressed according to conventional tabletting procedure using a suitably sized tabletting tooling. Tablets can be produced using conventional tabletting machines, and in the embodiments described below were produced on standards single punch F3 Manesty machine or Kilian RLE15 rotary tablet machine.

Generally speaking we find that even with highly water soluble active agents such as morphine or tramadol tablets formed by compression according to standard methods give very low in vitro release rates of the active ingredient e.g. corresponding to release over a period of greater than 24 hours, say more than 36. We have found that the in vitro release profile can be adjusted in a number of ways. For instance in the case of water soluble drugs a higher loading of the drug will be associated with increased release rates; the use of larger proportions of the water soluble fusible material in the particles or surface active agent in the tabletting formulation will also be associated with a higher release rate of the active ingredient: Thus, by controlling the relative amounts of these ingredients it is possible to adjust the release profile of the active ingredient, whether this be water soluble or water insoluble.

In Drug Development and Industrial Pharmacy, 20(7), 1179-1197 (1994) by L J Thomsen et al, which was published after the priority date of this application, a process similar to that described in PCT/SE93/00225 is discussed in detail. In the results and. discussion on page 1186 it is states that glyceryl monostearate was the only substance which showed pronounced potential as a meltable binder, and then only with mixers lined with polytetrafluoroethylene. By contrast the process of the present invention has been found to work satisfactorily with other binders and using conventional mixers with stainless steel linings.

In Pharmaceutical Technology Europe October 1994 pp 19-24 L J Thomsen describes the same process as mentioned in the above article. In the passage bridging pages 20 and 21 it is stated higher drug loads with larger drug crystals did not pelletize and that his results suggest manufacturers using melt pelletization should avoid starting materials containing amounts of crystals larger than 60µm and that electostatic charging of mass and adhesion to the walls of the mixer bowl made it impossible to make acceptable quality pellets with a binder of pure microcrystalline wax so that substantial amounts of glycerol monostearate was essential. In the process of the invention described herein drug crystal size has not been found to be a critical parameter; in the Examples described below the morphine sulphate typically has a particle size distribution with 50% of particles larger than 24 to 50µm and 10% larger than 100-140µm.

In order that the invention may be well understood the following examples are given by way of illustration only.

### EXAMPLES 1 to 4

Particles, having the formulations given in Table I below, were prepared by the steps of:
i) Placing the ingredients (a) to (c) (total batch weight 20kg) in the bowl of a 75 litre capacity Collette Vactron Mixer (or equivalent) equipped with variable speed mixing and granulating blades;
ii) Mixing the ingredients at about 150-350rpm whilst applying heat until the contents of the bowl are agglomerated.
iii) Classifying the agglomerated material by passage through a Comil and/or Jackson Crockatt to obtain controlled release particles.
iv) Adding the classified material to the heated bowl of a 75 litre Collette Vactron, allowing the particles to heat up under mixing, then adding ingredient (d), and continuing the mechanical working until uniform particles of the desired predetermined size range are formed in yields of greater than 80%.
v) Discharging the particles from the mixer and sieving them to separate out the particles collected between 0.5 and 2mm aperture sieves and then allowing them to cool.

**TABLE I**

| **EXAMPLE** | **1** | **2** | **3** |
|---|---|---|---|
| a) MORPHINE SULPHATE (WT%) B.P. | 55.0 | 52.19 | 53.48 |
| b) HYDROGENATED VEGETABLE OIL USNF (WT%) | 34.95 | 33.17 | 33.98 |
| c) POLYETHYLENE GLYCOL 6000 USNF (WT%) | 0.05 | 0.047 | 0.049 |
| d) HYDROGENATED VEGETABLE OIL USNF (WT%) | 10.0 | 14.60 | 12.49 |
| YIELD % | 90.5 | 83.4 | 90.1 |

The in vitro release rates of Examples 1, 2 and 3 were assessed by modified Ph. Eur. Basket method at 100 rpm in 900ml aqueous buffer (pH 6.5) containing 0.03%w/v polysorbate 80 at 37°C (corresponding to the Ph. Eur. Basket method but using a basket with a finer mesh, with the same open area and with a slightly concave top). For each of the products, six samples of the particles, each sample containing a total of 60mg of morphine sulphate were tested. The results set out in Table II below give the mean values for each of the six samples tested.

**TABLE II**

| **PRODUCT OF EXAMPLES** | | | |
|---|---|---|---|
| **HOURS AFTER START OF TEST** | **1** | **2** | **3** |
| | **% MORPHINE RELEASED** | | |
| 2 | 21 | 15 | 20 |
| 4 | 33 | 25 | 36 |
| 6 | 43 | 35 | 49 |
| 8 | 52 | 43 | 59 |
| 12 | 62 | 57 | 72 |
| 18 | 74 | 71 | 82 |
| 24 | 82 | 81 | 86 |
| 30 | 83 | 85 | 89 |

The procedure of Example 3 was repeated but the operation varied by adding the classified particles to a cold bowl of the Collette Vactron, followed by adding ingredient (d) and mixing, heating by jacket heating and microwave being applied during mixing. The in vitro release rate, obtained using the same procedure as for Examples 1 to 3, is given in Table IIa and demonstrates that although the composition of the products in Examples 3 and 4 are the same the different processing results in modified release rates.

**TABLE IIa**

| **PRODUCT OF EXAMPLE 4** | |
|---|---|
| **HOURS AFTER START OF TEST** | **% OF MORPHINE RELEASED** |
| 2 | 15 |
| 4 | 24 |
| 6 | 30 |
| 8 | 36 |
| 12 | 46 |
| 18 | 57 |
| 24 | 65 |
| 30 | 71 |

Particles produced according to Examples 1 to 4 were each blended with purified talc and magnesium stearate and used to fill hard gelatin capsules such that each capsule contains 60mg of morphine sulphate. The capsules produced were used in open, randomised crossover pharmacokinetic studies. As part of these studies patients received after overnight fasting either one capsule according to the invention or one MST CONTINUS^{R} tablet 30mg (a twice a day preparation). Fluid intake was unrestricted from 4 hours after dosing. A low-fat lunch was provided four hours after dosing, a dinner at 10 hours post dose and a snack at 13.5 hours post-dose. No other food was allowed until a 24 hour post-dose blood sample had been withdrawn. Blood samples were taken at the following times 1, 1.5, 2, 2.5, 3, 3.5, 4, 5, 6, 9, 12, 18, 24, 36, 48 and 72 hours post-dose.

The pharmacokinetic studies using these capsules gave peak plasma levels of from 3.2 to 29.2 ng/ml of morphine at median times between 2 and 6 hours following administration and blood sampling according to the above protocol.

The capsules containing particles produced according to Examples 2 and 4 in particular gave a mean Cmax of 11.9 ng/ml at median tmax 4 hours and mean Cmax of 9.2 nglml at median tmax 2.5 hours respectively (these values represent the mean of the individual Cmax and tmax values). In contrast the Cmax and tmax for the patients who received MST CONTINUS^{R} tablets were 10.6 -11.4 ng/ml and 2.0 -2.5 hours respectively. It was found, however, that the plasma concentrations of morphine in the blood of patients given capsules according to the invention at 24 hours were greater than the concentrations at 12 hours in those patients given MST CONTINUS tablets.

### Example 5

Particles were produced analogously to Examples 1 to 4 but having the following ingredients

| | **wt%** |
|---|---|
| Morphine sulphate | 55.0 |
| Hydrogenated vegetable oil | 44.7 |
| Polyethylene glycol 6000 | 0.3 |

Samples of the particles were then blended with magnesium stearate and purified talc in two lots (1 and 2) using a Y-Cone or bin-blender machine. The blended mixtures were then each compressed on a 7.1mm diameter normal concave tooling on a single punch F3 Manesty tabletting machine. The ingredients per dosage unit amounted to the following:

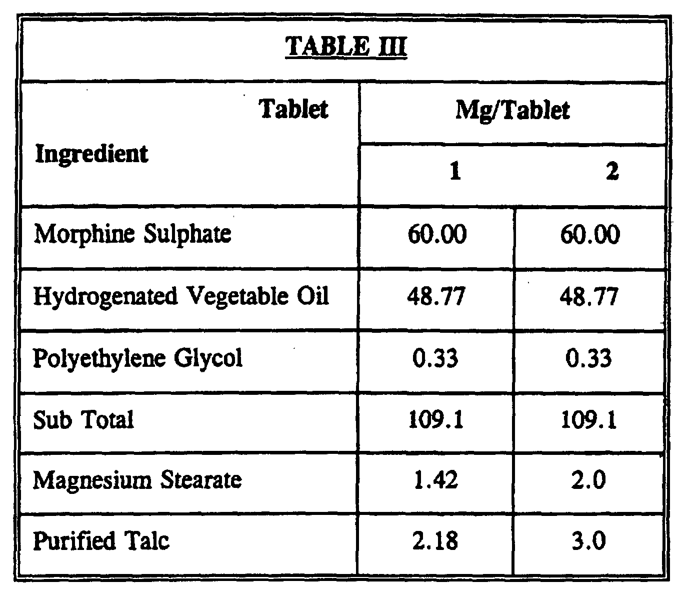

The dissolution of the samples of non-compressed particles (each sample containing 60mg of morphine sulphate) was assessed by the modified Ph. Eur. Basket method described above. For the dissolution of the tablets the Ph. Eur. Basket was replaced by the Ph. Eur. Paddle Method. The results are shown in Table IV below:

**TABLE IV**

| **HOURS AFTER START OF TEST** | **PARTICLES** | **TABLET 1** | **TABLET 2** |
|---|---|---|---|
| | **% MORPHINE SULPHATE RELEASED** | | |
| 1 | 27 | 13 | 11 |
| 2 | 43 | 20 | 17 |
| 4 | 63 | 29 | 26 |
| 8 | 82 | 42 | 37 |
| 12 | 88 | 50 | 44 |
| 16 | 91 | 57 | NR |
| 24 | 93 | 65 | NR |
| 30 | 94 | 70 | NR |
| 36 | 95 | 74 | NR |
| NR = Not recorded | | | |

The above results show that the tabletting procedure results in a considerable reduction in the release rate of the active ingredient.

### Example 6

The procedure of Example 5 was repeated but with the following variations.

The particles were made with the following ingredients.

| | **wt%** |
|---|---|
| Morphine Sulphate | 55.0 |
| Hydrogenated Vegetable Oil | 44.4 |
| Polyethylene Glycol 6000 | 0.6 |

Two lots of tablets (3 and 4) were produced from the particles using a 7.1mm diameter concave tooling. The ingredients per dosage unit were as follows;

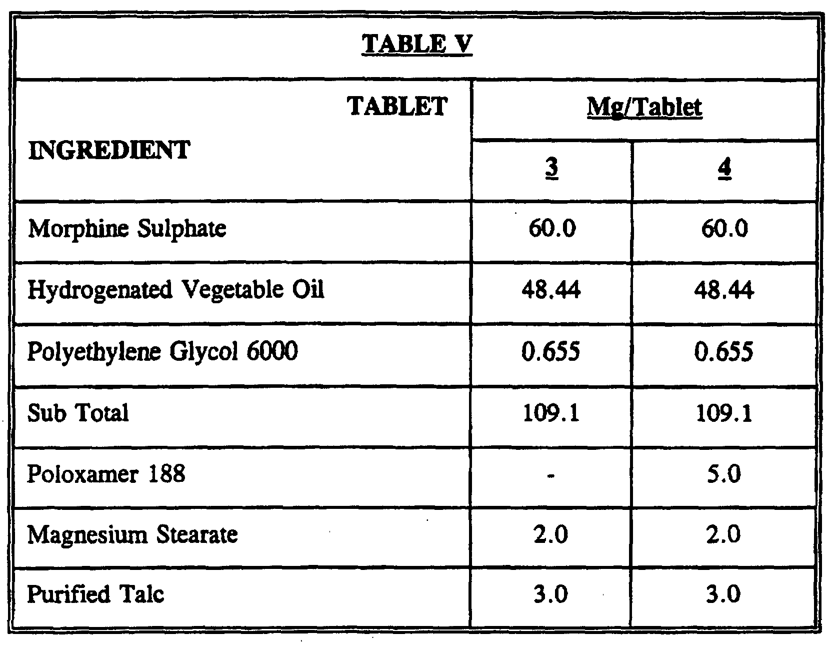

The dissolution of the tablets and samples of non-compressed particles (each sample containing 60mg of morphine sulphate) were assessed by the methods described above. The results are shown in Table VI below;

**TABLE VI**

| **HOURS AFTER START OF TEST** | **PARTICLES** | **TABLET 3** | **TABLET 4** |
|---|---|---|---|
| | **% MORPHINE SULPHATE RELEASED** | | |
| 1 | 56 | 16 | 19 |
| 2 | 75 | 24 | 28 |
| 4 | 90 | 34 | 38 |
| 8 | 95 | 46 | 52 |
| 12 | 97 | 54 | 60 |
| 16 | NR | NR | 67 |
| 24 | NR | NR | 77 |

These results demonstrate again a dramatic reduction in the release rate of the morphine sulphate resulting from compression tabletting of the particles; comparison of the release rates for Tablets 3 and 4 also show that the release rate can be adjusted by use of a surface active agent (in this case Poloxamer 188® ) as a tabletting excipient, the release rate for tablet 4 which contains the surface active agent being greater than that for tablet 3 without the surface active agent.

### Example 7

A procedure analogous to Example 5 was carried out using tramadol hydrochloride as active ingredient in place of morphine sulphate. The particles were made with the following ingredients;

| | **wt%** |
|---|---|
| Tramadol Hydrochloride | 50 |
| Hydrogenated Vegetable Oil | 50 |

Three lots of tablets (5, 6 and 7) were produced from particles using respectively (a) 14mm x 6mm, (b) 16mm x 7mm and (c) 18.6 x 7.5mm capsule shaped tooling. The ingredients per dosage unit were as follows;

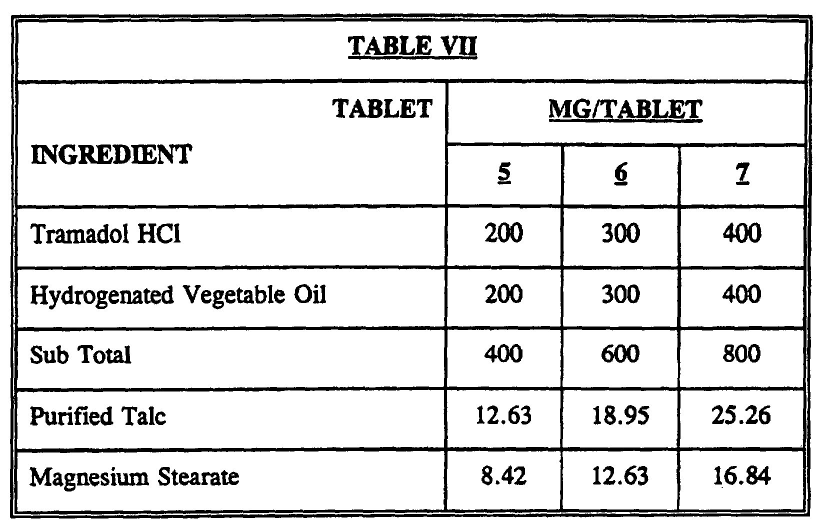

The tablets were assessed by dissolution in 0.1N HCl Ph. Eur. Paddle at 100rpm. For the non-compressed particles the Ph. Eur. Paddle was replaced by the modified Ph. Eur. Basket, each sample of particles containing 400mg of tramadol hydrochloride. The results are shown in Table VIII below;

| **HOURS AFTER START OF TEST** | **Particles** | **Tablet 5** | **Tablet 6** | **Tablet 7** |
|---|---|---|---|---|
| | **% TRAMADOL HCl RELEASED** | | | |
| 1 | 54 | 16 | 15 | 15 |
| 2 | 68 | 23 | 20 | 21 |
| 3 | 76 | 28 | 25 | 25 |
| 4 | 82 | 32 | 28 | 28 |
| 6 | 89 | 40 | 35 | 35 |
| 8 | 93 | 46 | 41 | 40 |
| 10 | 96 | 50 | 45 | 45 |
| 12 | 98 | 55 | 49 | 49 |
| 16 | 100 | 63 | 57 | 56 |
| 20 | NR | 70 | 63 | NR |

These results confirm the effectiveness of the tabletting in reducing the release rate of tramadol, a highly water soluble drug.

### Example 8

The procedure of Example 7 was repeated but with a higher loading of tramadol hydrochloride in the particles. Thus particles were made with the following ingredients;

| | **wt%** |
|---|---|
| Tramadol Hydrochloride | 75 |
| Hydrogenated Vegetable Oil | 25 |

Three lots of tablets (8, 9 and 10) were produced from the particles using respectively tooling (a), (b) and (c) described in Example 7. The ingredients per unit dosage were as follows:

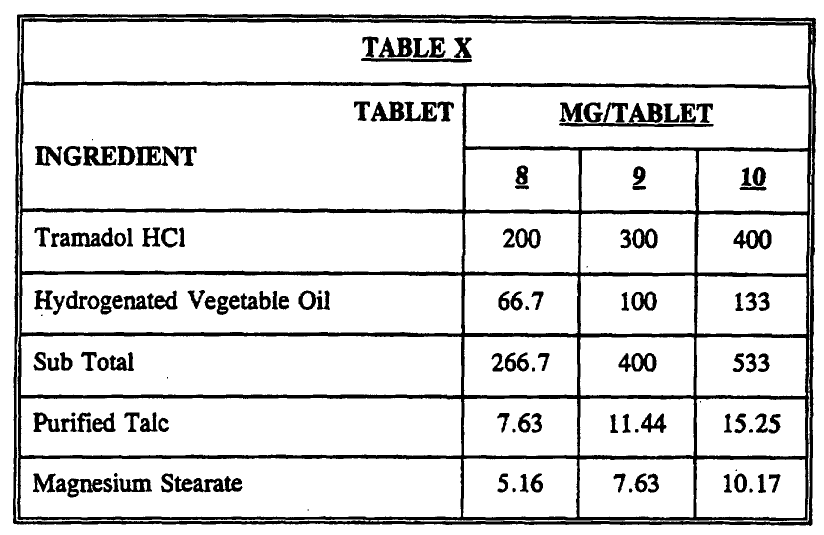

The tablets and samples of non-compressed particles (each sample containing 400mg of tramadol hydrochloride) were assessed by the methods described in Example 7. The results are shown in Table XI below;

| **HOURS AFTER START OF TEST** | **Particles** | **Tablet 8** | **Tablet 9** | **Tablet 10** |
|---|---|---|---|---|
| | **% TRAMADOL HCl RELEASED** | | | |
| 1 | 77 | 43 | 40 | 42 |
| 2 | 92 | 64 | 55 | 56 |
| 3 | 98 | 75 | 65 | 66 |
| 4 | 100 | 83 | 72 | 73 |
| 6 | 102 | 94 | 83 | 84 |
| 8 | 102 | 100 | 91 | 91 |
| 10 | 102 | NR | 96 | 97 |

These results show that by increasing the loading of the highly water soluble tramadol hydrochloride (75% w/w in this example compared with 50% w/w in Example 7) a significantly faster release rate of the active ingredient can be achieved.

### Example 9

0.35kg particulate diamorphine hydrochloride and the same weight of particulate hydrogenated vegetable oil (Lubritab) were placed in the bowl of a Collette Gral 10 or equivalent mixer, preheated to 60°C. Mixing was carried out at the following speeds for the Collette Gral 10 - mixer 350 rpm; chopper 1500 rpm, until the contents of the bowl are slightly agglomerated. The agglomerates are then allowed to cool to approximately 40°C, are removed from the bowl and are milled in a Comill to obtain controlled release seeds. The seeds are then placed in the mixer bowl and processing carried out until multiparticulates of a desired size are obtained. The contents of the bowl are then discharged and sieved to collect the 0.5 - 2.0mm sieve fraction.

The procedure described in the preceding paragraph was repeated but the collected sieve fraction is blended in a conventional blender with 0.006kg talc for 5 minutes; 0.004kg magnesium stearate is then added and the blending continued for 3 minutes. The blend is then discharged and compressed using a 4mm x 8mm capsule shaped tooling on a F3 tablet machine. The resulting tablet had a hardness of 1.7kp, a thickness of 2.8 - 3.0mm and a friability of < 1.0% and the following constitution:

| **CONSTITUENT** | **MG/TABLET** | **% W/W** |
|---|---|---|
| Diamorphine Hydrochloride | 40.0 | 47.6 |
| Hydrogenated Vegetable Oil | 40.0 | 47.6 |
| Talc | 2.40 | 2.86 |
| Magnesium Stearate | 1.6 | 1.91 |
| TOTAL | 84 | |

The dissolution rates of the resulting multiparticulates and tablets, measured respectively by the Ph. Eur. Basket or Paddle method at 100rpm in either phosphate or acetate buffer, were as follows:

| **TIME (HRS)** | **% DIAMORPHINE HCL RELEASED** | | |
|---|---|---|---|
| | **Multiparticulates Basket/Phosphate Buffer** | **Tablets Paddle/Phosphate Buffer** | **Tablets Paddle/Acetate Buffer** |
| 1 | 30 | - | 24 |
| 2 | 44 | 35 | 35 |
| 3 | 54 | 41 | 43 |
| 4 | 62 | 47 | 49 |
| 6 | 70 | 57 | 59 |
| 8 | 78 | 64 | 67 |
| 12 | 87 | 75 | 78 |
| 16 | 92 | 84 | 86 |

## Claims

1. A process for the manufacture of sustained release particles, which comprises
(a) mechanically working in a high-speed mixer, a mixture of a particulate drug and a particulate, hydrophobic and/or hydrophilic fusible carrier or diluent having a melting point from 35 to 150°C and optionally a release control component comprising a water-soluble fusible material or a particulate, soluble or insoluble organic or inorganic material, at a speed and energy input which allows the carrier or diluent to melt or soften whereby it forms agglomerates;
(b) removing agglomerates from the mixer and comminuting them to give controlled release particles; and optionally
(c) continuing mechanically working optionally with the addition of carrier or diluent in an amount of between 5% and 75% w/w of the total amount of ingredients; and optionally
(d) repeating steps (c) and possibly (b) one or more times.

2. A process according to claim 1, wherein during the mechanical working, heat is supplied thereto by microwave radiation.

3. A process according to claim 2, wherein only part of the heating is supplied by microwave radiation.

4. A process according to any one of claims 1 to 3, wherein the drug is morphine tramadol, hydromorphone, oxycodone, diamorphine or a pharmaceutically acceptable salt of any one of these.

5. A process according to any one of claims I to 4, wherein the hydrophobic fusible carrier(s) or diluent(s) is a wax.

6. A process according to claim 5, wherein the wax is chosen from hydrogenated vegetable oil, hydrogenated castor oil, Beeswax, Carnauba wax, microcrystalline wax and glycerol monostearate.

7. A process according to any one of claims 1 to 6, wherein the water-soluble fusible material optionally included in the mixture in step (a) is PEG having a molecular weight of from 1000 to 20000, preferably 1000 to 6000, or a poloxamer.

8. A process according to any one of claims 1 to 7 wherein the fusible carrier or diluent is added stepwise during mechanical working.

9. A process according to any one of claims 1 to 8, which further comprises the step of compressing the sustained release particles obtained by the process of any one of claims 1 to 8 to produce a solid, unitary dosage form.

10. A process according to any one of claims 1 to 8, which further comprises the step of filling sustained release particles obtained by the process of any one of claims 1 to 8 into a capsule.

## Patentansprüche

1. Verfahren zur Herstellung von Teilchen mit verzögerter Wirkstoffabgabe, mit den folgenden Schritten:
(a) mechanische Verarbeitung eines Gemischs aus einem teilchenförmigen Arzneimittel und einem teilchenförmigen, hydrophoben und/oder hydrophilen schmelzbaren Träger oder Streckmittel mit einem Schmelzpunkt von 35°C bis 150°C und wahlweise einer Abgabesteuerungskomponente mit einem wasserlöslichen schmelzbaren Material oder einem teilchenförmigen, löslichen oder unlöslichen, organischen oder anorganischen Material in einem Schnellmischer bei einer Geschwindigkeit und einer Energieaufnahme, die das Schmelzen oder Erweichen des Trägers oder Streckmittels ermöglicht, wodurch diese Agglomerate bilden;
(b) Entnahme der Agglomerate aus dem Mischer und Zerkleinern der Agglomerate zu Teilchen mit kontrollierter Wirkstoffabgabe; und wahlweise
(c) Fortsetzen der mechanischen Verarbeitung, wahlweise mit Zugabe eines Trägers oder Streckmittels in einem Anteil zwischen 5 Gew.-% und 75 Gew.-% der Gesamtmenge der Bestandteile; und wahlweise
(d) ein- oder mehrmaliges Wiederholen der Schritte (c) und möglicherweise (b).

2. Verfahren nach Anspruch 1, wobei während der mechanischen Verarbeitung Wärme durch Mikrowellenstrahlung zugeführt wird.

3. Verfahren nach Anspruch 2, wobei nur ein Teil der Erwärmung durch Mikrowellenstrahlung erfolgt.

4. Verfahren nach einem der Ansprüche 1 bis 3, wobei das Arzneimittel Morphin, Tramadol, Hydromorphon, Oxycodon, Diamorphin oder ein pharmazeutisch akzeptierbares Salz irgendeiner dieser Substanzen ist.

5. Verfahren nach einem der Ansprüche 1 bis 4, wobei der (die) hydrophobe(n) Träger oder das (die) Streckmittel ein Wachs ist.

6. Verfahren nach Anspruch 5, wobei das Wachs unter hydriertem Pflanzenöl, hydriertem Rizinusöl, Bienenwachs, Carnaubawachs, mikrokristallinem Wachs und Glycerinmonostearat ausgewählt ist.

7. Verfahren nach einem der Ansprüche 1 bis 6, wobei das wahlweise in dem Gemisch im Schritt (a) enthaltene wasserlösliche schmelzbare Material Polyethylenglycol (PEG) mit einem Molekulargewicht von 1000 bis 20000, vorzugsweise von 1000 bis 6000, oder ein Poloxamer ist.

8. Verfahren nach einem der Ansprüche 1 bis 7, wobei der schmelzbare Träger oder das schmelzbare Streckmittel während der mechanischen Verarbeitung schrittweise zugesetzt wird.

9. Verfahren nach einem der Ansprüche 1 bis 8, das ferner den Schritt zum Verdichten der Teilchen mit verzögerter Wirkstoffabgabe aufweist, die nach dem Verfahren nach einem der Ansprüche 1 bis 8 gewonnen werden, um eine feste, einheitliche Dosierungsform herzustellen.

10. Verfahren nach einem der Ansprüche 1 bis 8, das ferner den Schritt zum Einfüllen der nach dem Verfahren nach einem der Ansprüche 1 bis 8 gewonnenen Teilchen mit verzögerter Wirkstoffabgabe in eine Kapsel aufweist.

## Revendications

1. Procédé pour la fabrication de particules à libération prolongée, qui consiste:
(a) à travailler mécaniquement, dans un mélangeur à vitesse élevée, un mélange d'un médicament particulaire et d'un excipient ou d'un diluant particulaire, filsible, hydrophobe et/ou hydrophile présentant un point de filsion de 35 à 150°C et éventuellement un composant de contrôle de la libération comprenant une matière fusible soluble dans l'eau ou une matière organique ou inorganique, particulaire, soluble ou insoluble, à une vitesse et à un apport d'énergie qui permettent à l'excipient ou au diluant de fondre ou de ramollir, en conséquence de quoi il forme des agglomérats;
(b) à retirer les agglomérats du mélangeur et à les réduire en poudre pour donner des particules à liberation contrôlée; et éventuellement
(c) à poursuivre le travail mécanique avec éventuellement l'ajout d'un excipient ou d'un diluant en une quantité comprise entre 5% et 75% en poids de la quantité totale d'ingrédients; et éventuellement
(d) à répéter les étapes (c) et éventuellement (b) une fois ou plus.

2. Procédé suivant la revendication 1, dans lequel, durant le travail mécanique, de la chaleur est alimentée vers celui-ci par un rayonnement micro-onde.

3. Procédé suivant la revendication 2, dans lequel il n'y a qu'une partie du chauffage qui est alimentée par un rayonnement micro-onde.

4. Procédé suivant l'une quelconque des revendications 1 à 3, dans lequel le médicament est de la morphine, du tramadol, de l'hydromorphone, de l'oxycodone, de la diamorphine ou un sel pharmaceutiquement acceptable de n'importe lequel de ces composés.

5. Procédé suivant l'une quelconque des revendications 1 à 4, dans lequel le ou les excipients ou le ou les diluants fusibles hydrophobes sont une cire.

6. , Procédé suivant la revendication 5, dans lequel la cire est choisie parmi une huile végétale hydrogénée, de l'huile de ricin hydrogénée, de la cire d'abeilles, de la cire de Camauba, une cire microcristalline et du monostéarate de glycérol.

7. Procédé suivant l'une quelconque des revendications 1 à 6, dans lequel la matière fusible soluble dans l'eau éventuellement incluse dans le mélange dans l'étape (a) est du PEG possédant un poids moléculaire de 1000 à 20000, de préférence de 1000 à 6000, ou un poloxamère.

8. Procédé suivant l'une quelconque des revendications 1 à 7, dans lequel l'excipient ou le diluant fusible est ajouté progressivement durant le travail mécanique.

9. Procédé suivant l'une quelconque des revendications 1 à 8, qui comprend en outre l'étape de compression des particules à libération prolongée obtenues par le procédé suivant l'une quelconque des revendications 1 à 8 pour produire une forme de dosage unitaire solide.

10. Procédé suivant l'une quelconque des revendications 1 à 8, qui comprend en outre l'étape de remplissage des particules à libération prolongée obtenues par le procédé suivant l'une quelconque des revendications 1 à 8 dans une capsule.
